(19) Europäisches Patentamt
European Patent Office
Office européen des brevets

(11) EP 2 645 279 A2

(12) **EUROPEAN PATENT APPLICATION**

(43) Date of publication:
02.10.2013 Bulletin 2013/40

(51) Int Cl.:
*G06F 19/00* *(2011.01)*

(21) Application number: 12197764.9

(22) Date of filing: 18.12.2012

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME

(30) Priority: 29.03.2012 JP 2012078437

(71) Applicant: FUJITSU LIMITED
Kawasaki-shi,
Kanagawa 211-8588 (JP)

(72) Inventors:
• Matsuura, Azuma
  Kawasaki-shi, Kanagawa 211-8588 (JP)
• Satou, Hiroyuki
  Kawasaki-shi, Kanagawa 211-8588 (JP)

(74) Representative: Ward, James Norman
Haseltine Lake LLP
Lincoln House, 5th Floor
300 High Holborn
London WC1V 7JH (GB)

(54) **A program, apparatus, and method for determining bond types between atoms**

(57) A determination program causes a computer to execute a process that includes calculating by referring to a first storing unit storing an electron density of electrons belonging to each atom in a molecule and a degree of overlap of atomic orbitals between the atoms in the molecule, an electron density between a first atom and a second atom different from the first atom respectively selected from the molecule in a structurally stable state; determining a bond type of a bond between the first and the second atoms, based on the calculated electron density and by referring to a second storing unit correlating and storing bond types representing types of bonds between atoms, and conditions for the electron density between atoms for each bond type; and outputting the determined bond type between the first and the second atoms.

FIG.1

## Description

### FIELD

The embodiment discussed herein is related to determining a molecular structure.

### BACKGROUND

Conventionally, technologies that determine the structure of a molecule are known. In determining the bond form of a molecule, related technologies include, for example, a technique of displaying a molecular structure by selecting a bond form estimated from the bond order; and a technique of producing a trial structure to quickly acquired a proper molecular structure model in executing modeling for an amorphous- net polymer molecule in executing molecular simulation.

As for a method of determining the atomic species of two atoms in a molecule and a bonding type that represents the type of bond that combines the two atoms, based on the difference in the bonding state between the two atoms, a technique is present of determining the atomic species and the bond type using the atomic valence of each atom. For a molecular force field allocation method of allocating a molecular force field to a molecule having a molecular structure, a technique is present of determining the bond type by determining whether a predetermined threshold value is exceeded by the bonding distance between atoms that is calculated using quantum-scientific calculation referred to as "molecular orbital method" (see, e.g., Japanese Laid-Open Patent Publication Nos. H07-282096 and 2006-282929; Published Japanese-Translation of PCT Application, Publication No. 2008-041304WO; Wang, Junmei, et al, "Automatic Atom Type and Bond Type Perception in Molecular Mechanical Calculations", Journal of Molecular Graphics and Modeling, Vol. 25, 2006, pp. 247-260; and Fujitani, Hideaki, et al, "Massively Parallel Computation of Absolute Binding Free Energy with Well-Equilibrated States", Physical Review E, Vol. 79, 2009, 021914).

However, in the conventional techniques, the bonding distance between atoms can be acquired using various calculation methods of the quantum- scientific calculation, and the calculation results of the bonding distance are dispersed by the difference in the calculation method. Therefore, when the bond type between atoms is determined using the bonding distance of the atoms, the accuracy may be degraded in determining the bond type between the atoms.

### SUMMARY

It is an object in one aspect of the embodiments to at least solve the above problems in the conventional technologies.

A determination program causes a computer to execute a process that includes calculating by referring to a first storing unit storing an electron density of electrons belonging to each atom in a molecule and a degree of overlap of atomic orbitals between the atoms in the molecule, an electron density between a first atom and a second atom different from the first atom respectively selected from the molecule in a structurally stable state; determining a bond type of a bond between the first and the second atoms, based on the calculated electron density and by referring to a second storing unit correlating and storing bond types representing types of bonds between atoms, and conditions for the electron density between atoms for each bond type; and outputting the determined bond type between the first and the second atoms.

According to an aspect of the present invention, the accuracy in determining the bond type between atoms is improved.

### BRIEF DESCRIPTION OF DRAWINGS

FIG. 1 is an explanatory diagram of an example of operation of a determining apparatus according to an embodiment;

FIG. 2 is a block diagram of a hardware configuration of the determining apparatus according to the embodiment;

FIG. 3 is a block diagram of an example of a functional configuration of the determining apparatus;

FIG. 4 is an explanatory diagram of an example of the storage contents of a bond type determination condition table;

FIGs. 5A and 5B are explanatory diagrams of an example of the storage contents of a molecular structure table;

FIGs. 6A, 6B, and 6C are explanatory diagrams of an example of generation of initial values of a molecular structure by modeling software;

FIGs. 7A, 7B1, and 7B2 are explanatory diagrams of examples of a determination method for bond type using electron density;

FIGs. 8A1, 8A2, 8B1, and 8B2 are explanatory diagrams of examples of a determination method for an aromatic bond, executed using electron density;

FIGs. 9A and 9B are explanatory diagrams of an example of a determination method for a bond type having a charge, executed using electron density;

FIGs. 10A and 10B are explanatory diagrams of examples of a determination result concerning atomic species;

FIG. 11 is an explanatory diagram of an example of a comparison between a calculation result of electron density and a calculation result of bonding distance;

FIG. 12 is a flowchart of an example of a force field allocation process procedure;

FIG. 13 is a flowchart of an example of a bond type determination process procedure;

FIG. 14 is a flowchart of an example of an aromatic bond determination process procedure; and

FIG. 15 is a flowchart of an example of an anionic single bond determination process procedure.

DESCRIPTION OF EMBODIMENTS

Preferred embodiments of the present invention will be explained with reference to the accompanying drawings.

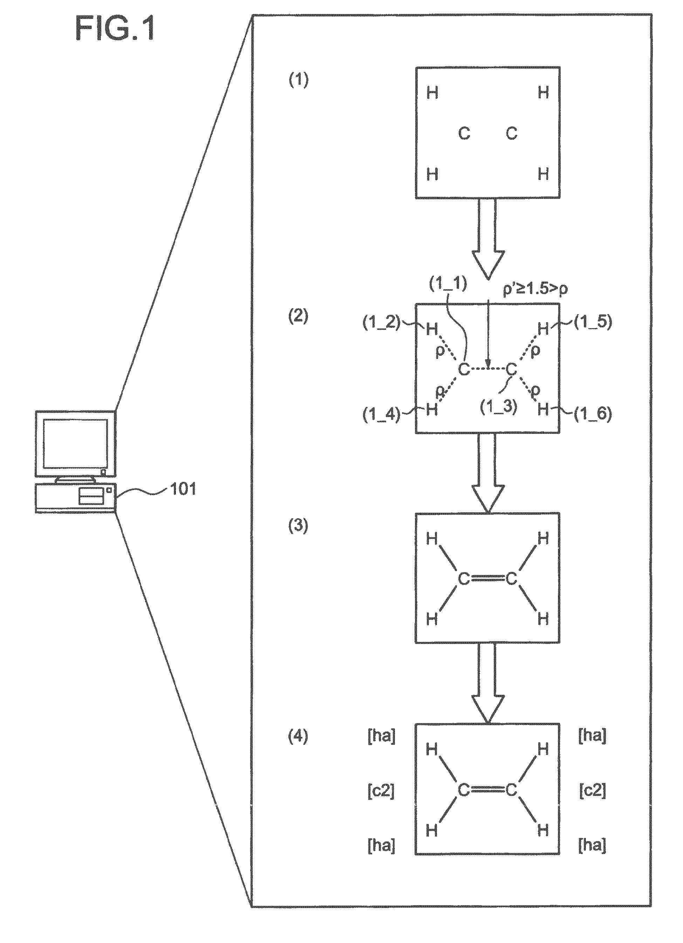

FIG. 1 is an explanatory diagram of an example of operation of the determining apparatus according to the embodiment. The operation example of the determining apparatus 101, which determines the bond type and allocates a molecular force field, will be described with reference to FIG. 1. When the determining apparatus 101 executes simulation for a new molecule or a new molecular aggregate, the determining apparatus 101 executes a process of assisting determination as to what molecular force field needs to be allocated to an atom or a bond between atoms included in a molecule. When the allocation of the molecular force field is improper, the accuracy of the simulation result is degraded and the result becomes unrealistic. The molecular force field can be uniquely identified based on the bond type between atoms and therefore, the determining apparatus 101 according to the embodiment is adapted to determine the proper bond type and thereby, allocate the proper molecular force field.

Molecular force field types include force fields related to electrostatic interaction energy, and bond types defined by the force field related to electrostatic interaction energy include, for example, a single bond, a double bond, a triple bond, an aromatic bond, a coordinate bond, and a delocalized bond. More detailed definitions of these bond types are described by Jakalian, Araz, et al, "Fast, Efficient Generation of High-Quality Atomic Charge, AM1-BCC Model: II, Parameterization and Validation", 2002, Journal of Computational Chemistry, Vol. 23, pp. 1623-1641. Hereinafter, the force field concerning electrostatic interaction energy will be referred to as "AM1BCC charge".

For example, force fields other than the AM1BCC charge include a general amber force field (GAFF). In the GAFF force field, a molecular force field can be uniquely allocated when the atomic species is determined. In determining the atomic species, the bond type is determined similarly to the AM1BCC charge. The GAFF force field is described by Wang, Junmei, et al, "Development and Testing of a General Amber Force Field", Journal of Computational Chemistry, Vol. 25, 2004, pp. 1157-1174. A force field corresponding to the GAFF atomic species is described by Cornell, Wendy D., et al, "A Second Generation Force Field for the Simulation of Proteins, Nucleic Acids, and Organic Molecules", Journals-American Chemical Society, Vol. 117, 1995, pp. 5179-5197.

An allocation approach of the GAFF will be described with reference to FIG. 1 taking an ethylene molecule whose chemical formula is C2H4 as an example. Hereinafter, each atom depicted with a reference numeral attached thereto in parentheses in FIGs. 1 to 15 will be expressed as "atom_(reference numeral)". For example, when a reference numeral "(1)" is attached to a hydrogen atom "H" in FIGs. 1 to 15, the hydrogen atom is expressed as "H_(1)". An atomic species is expressed using a character in brackets in FIGs. 1 to 15.

As depicted in FIG. 1, (1) it is assumed that a three-dimensional structure of an ethylene molecule is given to the determining apparatus 101 and that the three-dimensional structure is an energy-stable structure that is calculated using quantum-scientific calculation such as the molecular orbital method, density functional formalism, or a valence bond method.

As depicted in FIG. 1, (2) the determining apparatus 101 calculates the electron density between a first atom and a second atom (different from the first atom) that are selected from the ethylene molecule. The first and the second atoms may be a combination of any atoms. However, for example, atoms may be selected that are within a given distance such as, for example, a covalent radius, an ionic radius, or the Van der Waals radius. The "electron density between the two atoms" refers to the amount of electrons distributed per unit volume between the two atoms. In the embodiment, a charge density may be used instead of the electron density. The determining apparatus 101 executes the calculation using the electron density of the electrons belonging to each of the atoms in the ethylene molecule and the degree of overlap of atomic orbitals between the atoms in the ethylene molecule, the former and latter being parts of the calculation result of the quantum-scientific calculation, as the calculation method.

The determining apparatus 101 refers to conditions dictating the electron density between atoms in order for the atoms to be bonded by a given type of bond, and determines the bond type of the bond between the first and the second atoms based on the electron density calculated. For example, because the electron density p of C_(1_1) and H_(1_2) satisfies the electron density condition for a single bond (e.g., the electron density p is lower than 1.5), the determining apparatus 101 determines that the bond type of C_(1_1) and H_(1_2) is a single bond; and because the electron density ρ' of C_(1_1) and C_(1_3) satisfies the electron density condition for a double bond (e.g., electron density p' is greater than or equal to 1.5), the determining apparatus 101 determines that the bond type of C_(1_1) and C_(1_3) is a double bond.

The determining apparatus 101 determines that the bond type of C_(1_1) and H_(1_4), C_(1_3) and H_(1_5), and C_(1_3) and H_(1_6) are each single bonds. In FIG. 1, (3) the state after the determination of the bond type is depicted.

As depicted in FIG. 1, (4) because the bond types are determined, the determining apparatus 101 determines the atomic species from the bond types. The determining apparatus 101 determines that the atomic species of each of $C_{(1\_1)}$ and $C_{(1\_3)}$ is [c2] (sp2 carbon). The determining apparatus 101 determines that the atomic species of each of $H_{(1\_2)}$, $H_{(1\_4)}$, $H_{(1\_5)}$, and $H_{(1\_6)}$ is [ha] (hydrogen on aromatic carbon). Based on the above, when the determining apparatus 101 can determine the atomic species, the determining apparatus 101 can refer to the database of the molecular force field, can extract the corresponding molecular force field therefrom, and can allocate the molecular force field to the molecule.

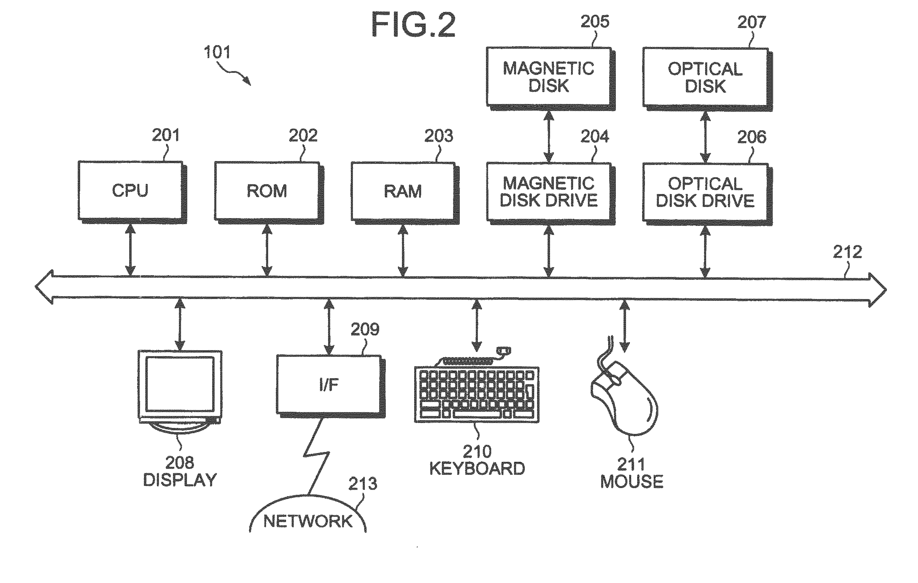

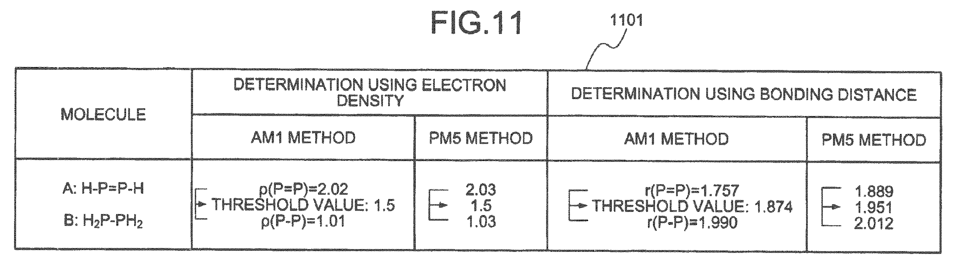

In this manner, irrespective of the quantum-scientific calculation method used to acquire the electron density, the determining apparatus 101 determines the bond type between atoms by calculating the electron density between the atoms, from the electron density of the atoms, which deviates minimally among the calculation methods. Thus, by standardizing the judgment criterion, the determining apparatus 101 prevents drops in accuracy caused by differences among the calculation methods. Calculation methods will be described later with reference to FIG. 11. The determining apparatus 101 will be described in detail with reference to FIGs. 2 to 15.

FIG. 2 is a block diagram of a hardware configuration of the determining apparatus according to the embodiment. As depicted in FIG. 2, the determining apparatus 101 includes a central processing unit (CPU) 201, a read-only memory (ROM) 202, and a random access memory (RAM) 203.

The determining apparatus 101 further includes a magnetic disk drive 204, a magnetic disk 205, an optical disk drive 206, and an optical disk 207, as well as a display 208, an interface (I/F) 209, a keyboard 210, and a mouse 211 as input/output devices for a user and external devices. The constituent components are respectively connected by a bus 212.

The CPU 201 governs overall control of the determining apparatus 101. The ROM 202 is non-volatile memory storing programs such as a boot program. The RAM 203 is used as a work area of the CPU 201. The magnetic disk drive 204, under the control of the CPU 201, controls the reading and writing of data with respect to the magnetic disk 205. The magnetic disk 205 stores therein data written under control of the magnetic disk drive 204.

The optical disk drive 206, under the control of the CPU 201, controls the reading and writing of data with respect to the optical disk 207. The optical disk 207 stores therein data written under control of the optical disk drive 206, the data being read by a computer. Any among the storage apparatuses, the ROM 202, the magnetic disk 205, and the optical disk 207 may store the determining program of the present embodiment.

The display 208 displays, for example, data such as text, images, functional information, etc., in addition to a cursor, icons, and/or tool boxes. A cathode ray tube (CRT), a thin-film-transistor (TFT) liquid crystal display, a plasma display, etc., may be employed as the display 208.

The I/F 209 is a control apparatus controlling an internal interface with a network 213 and controls the input/output of data from/to external apparatuses. The I/F 209 is connected to a network 213 such as a local area network (LAN), a wide area network (WAN), and the Internet through a communication line and is connected to other apparatuses through the network 213. For example, a modem or a LAN adaptor may be employed as the I/F 209.

The keyboard 210 includes, for example, keys for inputting letters, numerals, and various instructions and performs the input of data. Alternatively, a touch-panel-type input pad or numeric keypad, etc. may be adopted. The mouse 211 is used to move the cursor, select a region, or move and change the size of windows. A track ball or a joy stick may be adopted provided each respectively has a function similar to a pointing device.

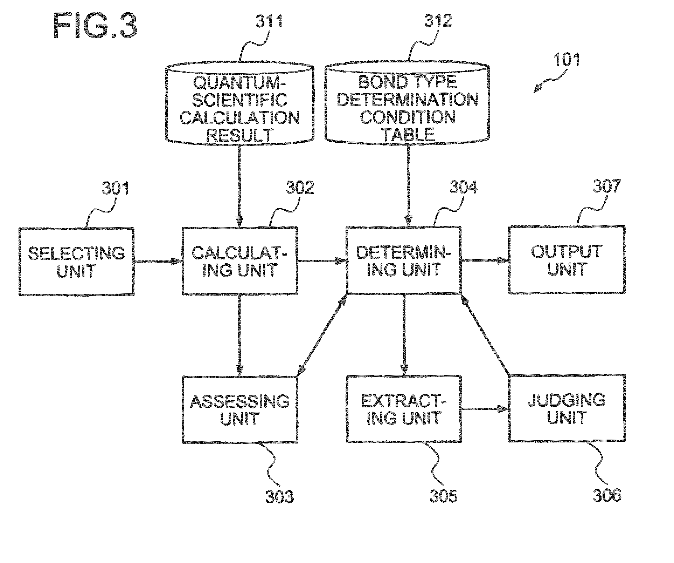

A functional configuration of the determining apparatus 101 will be described. FIG. 3 is a block diagram of an example of a functional configuration of the determining apparatus. The determining apparatus 101 includes a selecting unit 301, a calculating unit 302, an assessing unit 303, a determining unit 304, an extracting unit 305, a judging unit 306, and an output unit 307. Functions from the selecting unit 301 to the output unit 307 forming a control unit are implemented by causing the CPU 201 to execute programs stored in a storage apparatus. The storage apparatus is, for example, the ROM 202, the RAM 203, the magnetic disk 205, and the optical disk 207. Further, the functions may be implemented by an execution of the programs by another CPU through the I/F 209.

The determining apparatus 101 can access a quantum-scientific calculation result 311 that is a first storing unit and a bond type determination condition table 312 that is a second storing unit. The quantum-scientific calculation result 311 and the bond type determination condition table 312 are stored in a storage apparatus such as the RAM 203, the magnetic disk 205, and the optical disk 207.

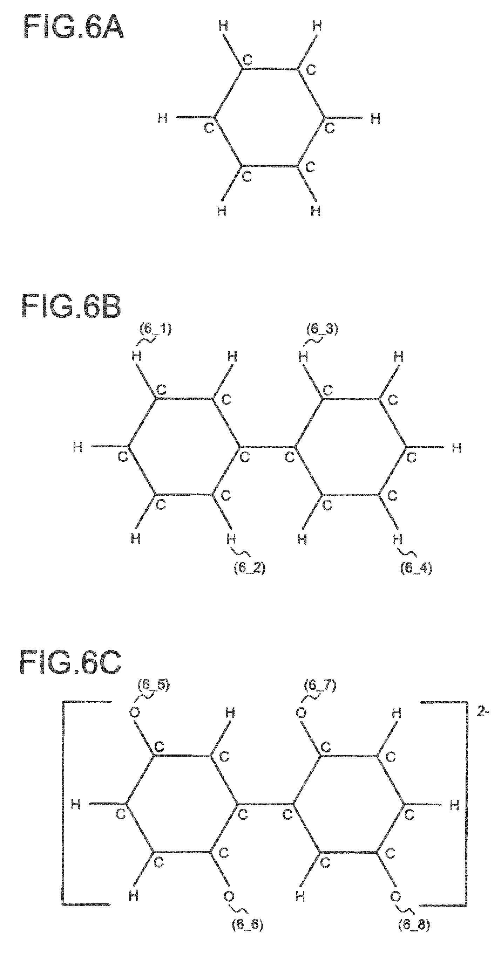

The quantum-scientific calculation result 311 stores the electron density of the electrons belonging to each atom in a molecule in a structurally stable state, and the degree of overlap of the atomic orbitals between the atoms in the molecule. The electron density of the electrons belonging to each of the atoms in the molecule in a structurally stable state is, for example, an electron density matrix. The degree of overlap of the atomic orbitals between atoms in the molecule is, for example, an overlap integral matrix. The electron density matrix and the overlap integral matrix will be described later with reference to FIG. 6.

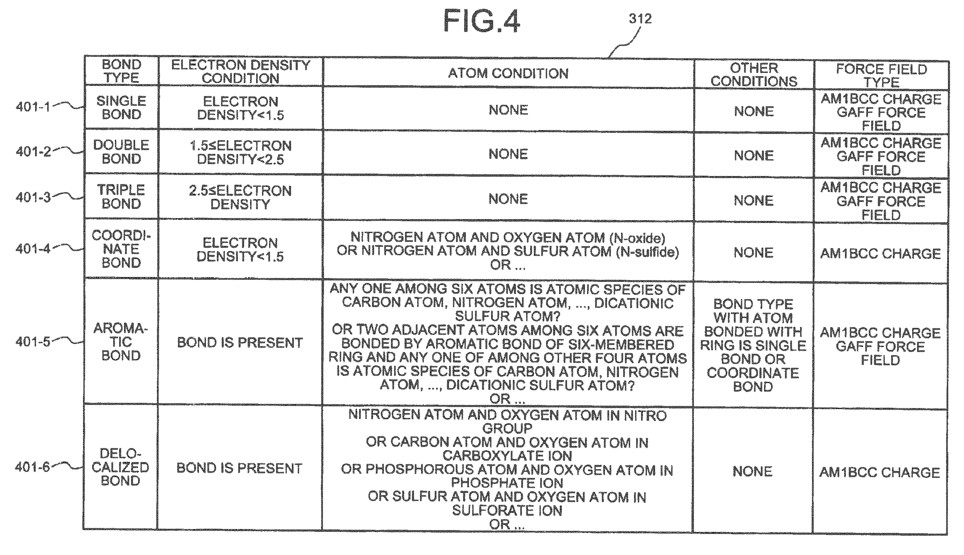

The bond type determination condition table 312 correlates and stores bond types that represent types of bonds between atoms, and conditions for the electron density between atoms for each bond type. The bond type determination condition table 312 may correlate and store with a single bond that represents a type of bond between atoms, a first condition for the electron density between atoms to be bonded by a single bond. The bond type determination condition table 312 may correlate and store with a double bond that represents a type of bond between atoms, a second condition for the electron density between atoms to be bonded by a double bond. The bond type determination condition table 312 may correlate and store with a triple bond that represents a type of bond between atoms, a third condition for the electron density between atoms to be bonded by a triple bond. Details of the first to the third conditions will be described with reference to FIG. 4.

The bond type determination condition table 312 may correlate and store with a coordinate bond that represents a type of bond between the atoms, a fourth condition for the electron, density between atoms bonded by the coordinate bond. The bond type determination condition table 312 may further correlate and store with the coordinate bond, a fifth condition for a combination of species of atoms bonded by a coordinate bond. Details of the fourth and the fifth conditions will be described with reference to FIG. 4. The atomic species refers to the kind of element. The atomic species may include the state of an electric charge such as being cationic, being neutral, and being anionic. Hereinafter, absence of description as to the state of an electric charge means being neutral.

The bond type determination condition table 312 may correlate and store with an aromatic bond that represents a type of bond between atoms, a sixth condition for the species of atoms capable of forming a ring by being bonded by an aromatic bond. Details of the sixth condition will be described with reference with FIG. 4.

The bond type determination condition table 312 may correlate and store with the aromatic bond, a seventh condition for the type of bond between a ring formed by atoms bonded with each other by aromatic bonds, and atoms bonded with the ring. Details of the seventh condition will be described with reference to FIG. 4.

The selecting unit 301 selects from the molecule, the first atom and the second atom that is different from the first atom. Taking an example of FIG. 1, the selecting unit 301 selects $C\_(1\_1)$ and $H\_(1\_2)$ from the ethylene molecule. The data selected is stored to a storage area such as the RAM 203, the magnetic disk 205, and the optical disk 207.

The calculating unit 302 refers to the bond type determination condition table 312 and calculates from the molecule, the electron density between the first and the second atoms selected by the selecting unit 301. The first and the second atoms may be two arbitrary atoms in the molecule, selected by user operation. For example, the calculating unit 302 calculates the electron density between the first and the second atoms using a method by Mulliken or Loewdin. The calculating unit 302 may use the bond order as the electron density. For example, the calculating unit 302 may use the bond order by Mayer or the bond order by Coulson. The bond order by Mayer is described in detail in Mayer, I., "Charge, Bond Order and Valence in the ab initio SCF Theory", Chemical Physics Letters, Vol. 97, 1983, pp. 270-274.

The bond order of Mayer shows a value of about 1, 1.5, 2, and 3, respectively, for a single bond, a conjugated bond, a double bond, and a triple bond. When no bond is present, the bond order of Mayer shows substantially zero. A detailed description thereof is made in Kalinowski, Jaroslaw A., et al, "Class IV Charge Model for the Self-Consistent Charge Density-Functional Tight-Binding Method", Journal of Physical Chemistry A, Vol. 108, 2004, pp. 2545-2549.

A case will be described where the bond order of Mayer is used. For example, the calculating unit 302 calculates the electron density between $C\_(1\_1)$ and $H\_(1\_2)$ as 1.01. The data calculated is stored to a storage area such as the RAM 203, the magnetic disk 205, and the optical disk 207.

The assessing unit 303 assesses which among the first, the second, and the third conditions stored in the bond type determination condition table 312 is satisfied by the electron density calculated by the calculating unit 302. For example, it is assumed that the electron density calculated is 1.01; and that the first condition is that the electron density<1.5, the second condition is that 1.5≤the electron density<2.5, and the third condition is that 2.5≤the electron density. In this case, the assessing unit 303 assesses that the electron density calculated satisfies the first condition.

The assessing unit 303 may assess whether the electron density calculated by the calculating unit 302 satisfies the fourth condition stored in the bond type determination condition table 312. For example, it is assumed that the electron density calculated is 1.01, and that the electron density<1.5. The assessing unit 303 assesses that the fourth condition is satisfied.

The assessing unit 303 may assess whether the combination of species of the first and the second atoms satisfies the fifth condition stored in the bond type determination condition table 312. For example, it is assumed that the species of the first atom is a nitrogen atom and the species of the second atom is an oxygen atom and that the fifth condition requires a nitrogen atom and an oxygen atom. The assessing unit 303 assesses that the combination of species of the first and the second atoms satisfies the fifth condition.

The assessing unit 303 may assess whether an atom group is present that forms a ring in the molecule. The ring is a structure formed by the atom group bonded that forms a ring shape. The ring is a three-membered ring when the number of atoms forming the ring is three, the ring is a five-membered ring when the number of atoms forming the ring is five, etc. An example of the method of identifying a state where atoms are bonded is a method where the atoms are identified as being bonded with each other if the electron density between the first and the second atoms does not stay in the vicinity of zero and is a value greater than or equal to a specific value. Another example of a method of identifying a state where the atoms are bonded is a method where the atoms are identified to be bonded with each other if the distance between the first and the second atoms is within a distance such as the covalent radius, the ionic radius, or the Van der Waals radius. For example, the assessing unit 303 assesses that the molecule includes six carbon atoms that form a six-membered ring.

When the assessing unit 303 assesses that an atom group is present that forms a ring, the assessing unit 303 may assess whether the species of each of the atoms of the atom group satisfies the sixth condition stored in the bond type determination condition table 312. For example, it is assumed that six carbon atoms are present that form a six-membered ring in the molecule; and that the sixth condition is that the species of the atoms forming the six-membered ring is any one of a carbon atom, a nitrogen atom, ..., a dicationic sulfur atom. In this example, the assessing unit 303 assesses that the species of each of the atoms of the atom group forming the six-membered ring satisfies the sixth condition.

When the determining unit 304 determines the type of bond between the first and the second atoms and the assessing unit 303 assesses that an atom group is present that forms a ring in the molecule, the assessing unit 303 may assess whether an atom is present that is bonded with the ring in the molecule. For example, it is assumed that the type of bond between an oxygen atom and a carbon atom has been determined to be a single bond; that the molecule includes a six-membered ring; and that the six-membered ring includes a carbon atom that is determined to be bonded by the single bond. In this example, the assessing unit 303 assesses that an oxygen atom is present as an atom bonded with the six-membered ring.

When the assessing unit 303 assesses that an atom is present that is bonded with the six-membered ring in the molecule, the assessing unit 303 may assess whether a seventh condition stored in the bond type determination condition table 312 is satisfied by the type of bond between the ring in the molecule and the atom bonded with the ring. For example, it is assumed that six carbon atoms are present that form a six-membered ring in the molecule, that the bond type of each of the atoms bonded with the six carbon atoms forming the six-membered ring is a single bond, and that the seventh condition is that the type of bond between the ring and the atom bonded with the ring is a single bond or a coordinate bond. In this example, the assessing unit 303 assesses that the seventh condition is satisfied by the type of bond between the six-membered ring and the atom bonded with the six-membered ring. The assessed data is stored in a storage area such as the RAM 203, the magnetic disk 205, and the optical disk 207.

The determining unit 304 refers to the bond type determination condition table 312 and determines the type of bond between the first and the second atoms based on the electron density calculated. For example, when the conditions of the records of the bond type determination condition are satisfied, the determining unit 304 determines that the bond type of the corresponding record is the type of bond between the first and the second atoms. When the assessing unit 303 assesses that any one of the first to the third conditions is satisfied, the determining unit 304 may determine that the type of bond between the first and the second atoms is the type of bond between the atoms correlated with the condition that is determined to be satisfied by the calculated electron density. For example, when the first condition is satisfied, the determining unit 304 determines that the type of bond between the first and the second atoms is a single bond, which is correlated with the first condition.

When the assessing unit 303 assesses that the fourth and the fifth conditions are satisfied, the determining unit 304 may determine that the type of bond between the first and the second atoms is a coordinate bond. When the assessing unit 303 assesses that the sixth condition is satisfied, the determining unit 304 may determine that the type of bond between the atoms of the atom group forming the ring is an aromatic bond. When the assessing unit 303 assesses that the seventh condition is satisfied, the determining unit 304 may determine that the type of bond between the atoms of the atom group forming the ring is an aromatic bond.

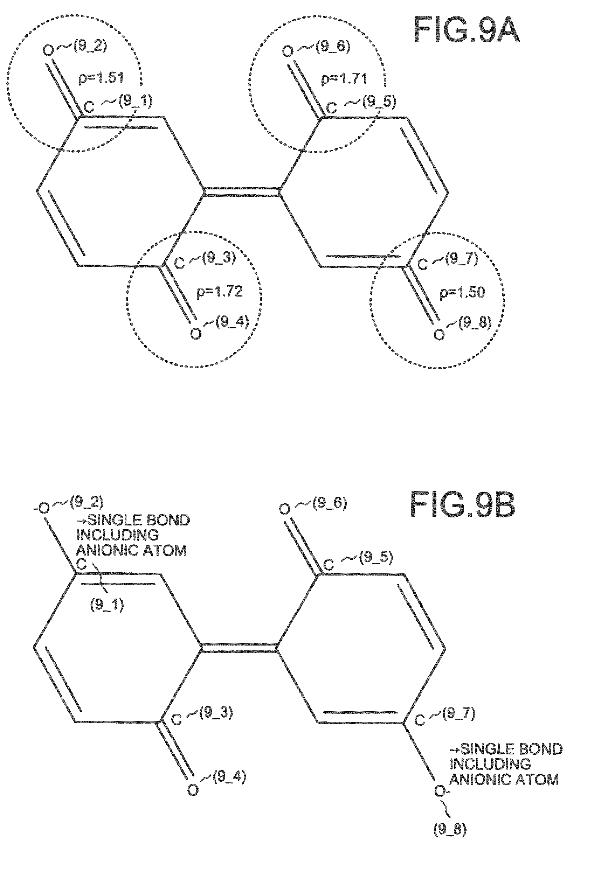

The determining unit 304 determines that the type of bond is a single bond, between the atoms whose electron density is the lower one of the electron density between the first and the second atoms, and the electron density between the third and the fourth atoms. The determining unit 304 determines that any one atom is an anionic atom, among the two atoms whose bond type is determined to be a single bond. The determining unit 304 executes when the judging unit 306 judges that the electron density between the first and the second atoms and the electron density between the third and the fourth atoms are not equal to each other. For example, the determining unit 304 determines that the atom whose number of atoms bonded with it is few compared to its valence is an anionic atom, among the two atoms. A process will be described with reference to FIG. 9. The data determined is stored to a storage area such as the RAM 203, the magnetic disk 205, and the optical disk 207.

The extracting unit 305 extracts, from the atom group that remains after excluding the first and the second atoms, the third and the fourth atoms that are bonded to each other by a double bond and whose atomic species combination is same as that of the first and the second atoms. The extracting unit 305 executes when the determining unit 304 determines that the type of bond between the first and the second atoms is a double bond. For example, it is assumed that the type of bond is a double bond between an oxygen atom regarded as the first atom and a carbon atom as the second atom. In this case, the extracting unit 305 extracts the third and the fourth atoms whose bond type is a double bond and that form a combination of an oxygen and a carbon atoms. The data extracted is stored to a storage area such as the RAM 203, the magnetic disk 205, and the optical disk 207.

The judging unit 306 judges whether the electron density between the first and the second atoms, and the electron density between the third and the fourth atoms extracted by the extracting unit 305 are equal to each other. The values of the electron densities do not need to be equal to each other for a judgment of equality of the electron densities. A judgment method will be described later with reference to FIG. 9. The judgment results are stored to a storage area such as the RAM 203, the magnetic disk 205, and the optical disk 207.

The output unit 307 outputs the type of bond between the first and the second atoms determined by the determining unit 304. The output unit 307 outputs the result to, for example, the display 208 as an output destination, or may output the result to a storage area such as the RAM 203, the magnetic disk 205, or the optical disk 207.

FIG. 4 is an explanatory diagram of an example of the storage contents of the bond type determination condition table. The depicted bond type determination condition table 312 stores data according to bond type; has records 401-1 to 401-6; and has five fields for "bond type", "electron density condition", "atom condition", "other conditions", and "force field type", respectively. The bond type field stores identification information that indicates the bond type. The electron density condition field stores a condition for the electron density between the atoms to be bonded by the bond type. The atom condition field stores the atomic species bonded by the bond type. The other conditions field stores conditions other than the electron density condition and the atom condition. The force field type field stores the type of the force field that has the atomic species defined therefor.

For example, the record 401-1 presents that the electron density needs to be less than 1.5 as a condition for the bond to be a single bond; and that a single bond is defined for both the AM1BCC charge and the GAFF force field. The contents of the electron density condition field of the record 401-1 is the first condition.

Similarly, the contents of the electron density condition field of the record 401-2 is the second condition; that of the record 401-3 is the third condition; that of the record 401-4 is the fourth condition; that of the record 401-5 is the fifth condition; and that of the record 401-6 is the sixth condition. Details of the sixth condition are described in the above '096 Publication with reference to FIG. 1a thereof. The contents of the atom condition field of the record 401-5 is the seventh condition. An example will be described of the storage contents of a molecular structure table 501 that stores the molecular structures.

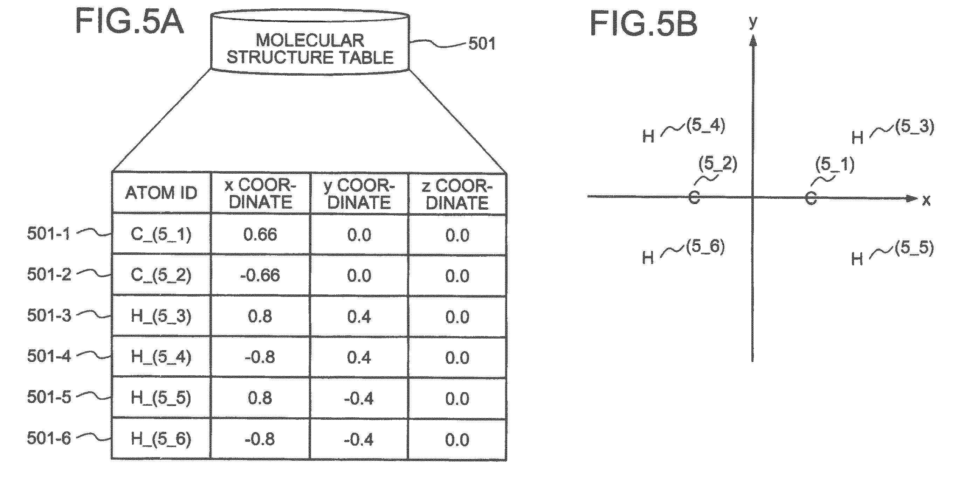

FIGs. 5A and 5B are explanatory diagrams of an example of the storage contents of the molecular structure table. The description will be made with reference to FIGs. 5A and 5B taking an example of a case where the molecular structure of an ethylene molecule is stored in the molecular structure table 501. FIG. 5A depicts an example of the storage contents of the molecular structure table 501. FIG. 5B depicts the positions of the atoms in an x-y coordinate system that corresponds to the storage contents of the molecular structure table 501.

The molecular structure table 501 depicted in FIG. 5A has records 501-1 to 501-6, and includes four fields for "atom ID", "x coordinate", "y coordinate", and "z coordinate", respectively. The atom ID field stores identification information to identify an atom to be handled. The x coordinate field stores an x-coordinate value of the atom. The y coordinate field stores a y-coordinate value of the atom. The z coordinate field has a z-coordinate value of the atom. For example, the record 501-1 presents that $C_{(5\_1)}$ is located at a position whose x coordinate is x=0.66, whose y coordinate is y=0.0, and whose z coordinate is z=0.0.

In FIG. 5B, $C_{(5\_1)}$ to $H_{(5\_6)}$ presented by the records 501-1 to 501-6 are depicted in the x-y coordinate system. Because the z-coordinate values of $C_{(5\_1)}$ to $H_{(5\_6)}$ are all zero in FIG. 5B, the z-axis is not depicted. Processes from generation of the molecular structure to allocation of molecular force fields for $C_{12}H_6O_4$ will be described with reference to FIGs. 6A to 10B.

FIGs. 6A, 6B, and 6C are explanatory diagrams of an example of generation of initial values of the molecular structure by modeling software. A state where data to be stored to the molecular structure table 501 is generated by the modeling software will be described with reference to FIGs. 6A, 6B, and 6C.

In FIG. 6A, the determining apparatus 101 displays a benzene ring consequent to a user operation. For example, on the determining apparatus 101, a benzene-ring icon on a tool bar is pressed down by a user operation via the mouse 211. The determining apparatus 101 displays thereon the benzene ring at a position in its working window by a clicking of the mouse 211 by the user.

In FIG. 6B, the determining apparatus 101 displays a second benzene ring consequent to a user operation via the mouse 211. The determining apparatus 101 displays a state where the first and the second benzene rings are bonded, consequent to a user operation via the mouse 211.

FIG. 6C is a diagram acquired by replacing $H_{(6\_1)}$ to $H_{(6\_4)}$ in (B) of FIG. 6 with $O_{(6\_5)}$ to $O_{(6\_8)}$ by a user operation via the mouse 211, on the determining apparatus 101. The determining apparatus 101 sets the electric charge of the entire molecule to be "-2" consequent to a user operation via the mouse 211. Using the molecular orbital method, a stable structure is acquired of $C_{12}H_6O_4$ generated by the operations depicted in FIGs. 6A, 6B, and 6C. A parametric method 5 (PM5) method belonging to a neglect of diatonic differential overlap (NDDO) method is used as the calculation method of the molecular orbital method.

The determining apparatus 101 calculates the bond order of Mayer of the portion having the bond, using the PM5 method. The determining apparatus 101 calculates the bond order of Mayer using Eq. (1) below.

$$BO_{kk'} = \sum_{\lambda \in k} \sum_{\omega \in k'} (PS)_{\omega\lambda} (PS)_{\lambda\omega}$$

$$(1)$$

In the above, "$BO_{kk'}$" represents the bond order of Mayer between an atom k and an atom k; "P" and "S" respectively represent an electron density matrix and an atomic orbital overlapping integral matrix of each electron; and "$\lambda$" and "$\omega$" respectively represents the basic functions belonging to k and k'. The density matrix P and the overlapping integral matrix S are executed when the energy is calculated in the quantum-scientific calculation and therefore, are calculations not executed wastefully when the bond order is acquired. Consequently, the amount of calculation for acquiring the bond order is negligibly small compared to that of the quantum-scientific calculation. The determining apparatus 101 calculates the matrix elements of the density matrix P and the overlapping integral matrix S according to Eqs. (2) and (3) below.

$$P_{\mu\nu} = 2\sum_{i}^{n} C_{\mu i} C_{\nu i}$$

$$(2)$$

$$S_{\mu\nu} = \int \chi_\mu \chi_\nu dv$$

$$(3)$$

In the above: "p" and "$\gamma$" are suffixes for the atomic orbitals; "$c_{\mu}i$" and "$c\gamma i$" represent an i-th lowest molecular orbitals of the potential energy that the molecule can take, or an orbital coefficient of the orbital in the density functional formalism; "$\chi\mu$" and "$\chi\gamma$" represent basic functions to develop the molecular orbitals (atomic orbitals); and "n" is number of orbitals that the electron occupies. For example, when 10 electrons are present, n is five. Representing the i-th orbital as "$\psi_i$", the relation among $\psi_i$, $c\mu_i$, and $\chi\mu$ is expressed in Eq. (4) below.

$$\psi_i = \sum_{\mu} C_{\mu i} \chi_\mu$$

$$(4)$$

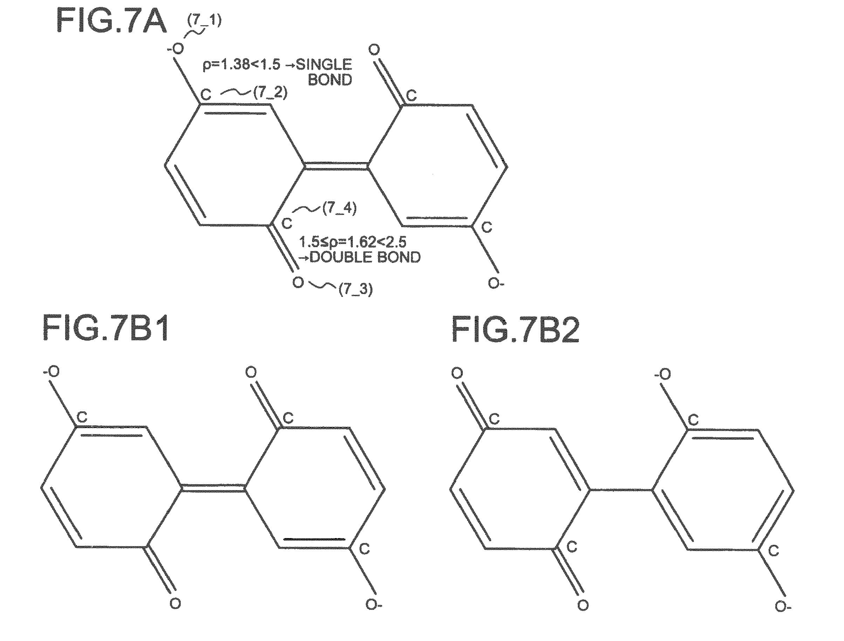

FIGs. 7A, 7B1, and 7B2 are explanatory diagrams of examples of a determination method for the bond type using electron density. FIG. 7A depicts the determination method for the bond type using electron density. FIGs. 7B1 and 7B2 depict the determination method for the bond type using valence.

According to the method that uses the electron density depicted in FIG. 7A, the determining apparatus 101 calculates a value of 1.38 for the electron density p between O_(7_1) and C_(7_2), and determines the bond type of O_(7_1) and C_(7_2) to be a single bond because the condition for the value of $\rho l$ indicating a single bond is satisfied, i.e., the electron density $\rho$ is $\rho<1.5$. Because the bond type is a single bond, the determining apparatus 101 determines that the oxygen atom is an anionic oxygen atom whose valence is one. It is assumed that information indicating that, when the valence of an oxygen atom is one, the oxygen atom is anionic and that, when its valence is two, the oxygen atom is neutral, are stored as a table in the RAM 203, the magnetic disk 205, or the optical disk 207.

The determining apparatus 101 calculates a value of 1.62 for the electron density $\rho$ between O_(7_3) and C_(7_4), and determines that the bond type of O_(7_3) and C_(7_4) is a double bond because the condition for the value of $\rho$ to indicate the double bond is satisfied, i.e., the electron density $\rho$ is $1.5 \leq \rho < 2.5$. In this manner, the determining apparatus 101 determines that the bond type is a single bond when the value of $\rho$ satisfies the condition $\rho<1.5$ and determines that the bond type is a double bond when the value of $\rho$ satisfies the condition $1.5 \leq \rho < 2.5$. Although not depicted in FIGs. 7A, 7B1, or 7B2, the determining apparatus 101 determines that the bond type is a triple bond when the value of $\rho$ satisfies the condition $2.5 \leq \rho$.

Because the valence of O⁻ is one and that of O is two, the method executed using the valence depicted in FIGs. 7B1 and 7B2 has two cases for a structure as depicted in FIG. 7B1 and in FIG. 7B2, and the determination of which structure is correct is difficult. As described, the method executed using the valence leaves ambiguity in the determination. The structures that are depicted in FIGs. 7A and 7B1 are stable as structures that may actually be taken and each diagonally has therein anionic oxygen atoms. Therefore, the determining apparatus 101 can more correctly determine the bond type by using the determination method executed using the electron density than by that executed using the valence.

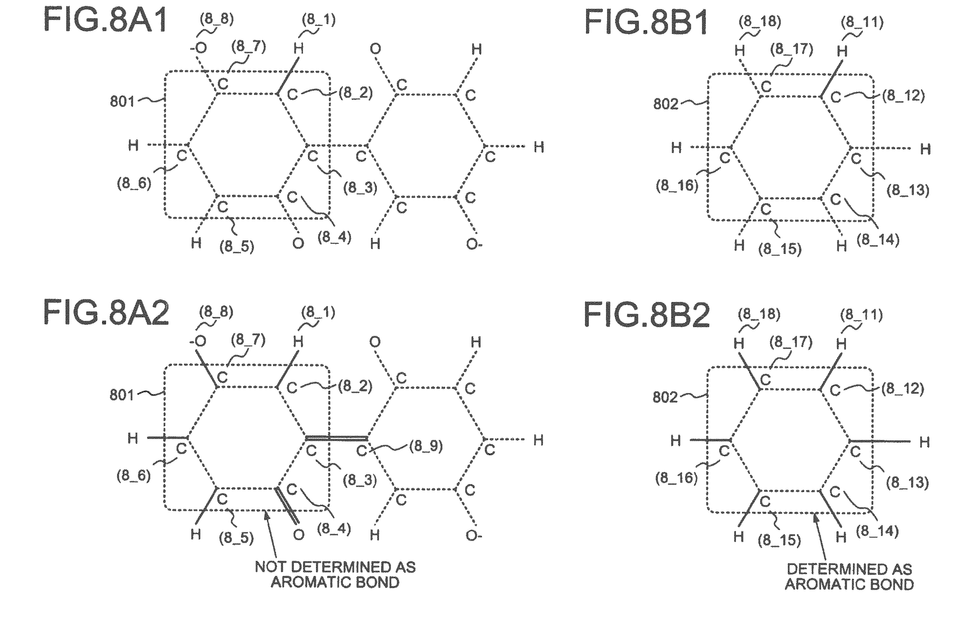

FIGs. 8A1, 8A2, 8B1, and 8B2 are explanatory diagrams of examples of the determination method for an aromatic bond, executed using electron density. The determination method for the type of bond between atoms included in a structure will be described with reference to FIGs. 8A1, 8A2, 8B1, and 8B2. For example, the determining apparatus 101 determines the type of bond between atoms included in $C_{12}H_6O_4$ in FIGs. 8A1 and 8A2, and determines the type of bond between atoms include in $C_6H_6$ in FIGs. 8B1 and 8B2.

It is assumed that, in FIG. 8A1, the determining apparatus 101 determines that the bond type between $H\_(8\_1)$ and $C\_(8\_2)$ is a single bond. The determining apparatus 101 determines whether any one among $H\_(8\_1)$ and $C\_(8\_2)$ is included in the ring. In the case of FIG. 8A1, $C\_(8\_2)$ is included in the ring because $C\_(8\_2)$ to $C\_(8\_7)$ form the ring. Hereinafter, the ring formed by $C\_(8\_2)$ to $C\_(8\_7)$ will be referred to as "ring 801". Because $C\_(8\_2)$ is included in the ring 801, the determining apparatus 101 determines whether the combination of atom groups forming the ring 801 is a specific combination. A specific combination refers to the atom condition of the record 401-5. In this case, the atom group forming the ring 801 includes only carbon atoms and this corresponds to the specific combination.

When the ring 801 is formed by the specific combination, the determining apparatus 101 determines whether the type of bond between the ring 801 and each of the atoms bonded with the ring 801 is a single bond or a coordinate bond. At the stage depicted in FIG. 8A1, the type of bond between the ring 801 and each of the atoms bonded with the ring 801, that is, the type of bond between, for example, $C\_(8\_7)$ and $O\_(8\_8)$ is not determined and therefore, the determining apparatus 101 does not determine whether the type of bond between the atoms included in the ring 801 is an aromatic bond.

It is assumed that, in FIG. 8A2, the determining apparatus 101 determines that the type of bond between $C\_(8\_7)$ and $O^-\_(8\_8)$ is a single bond, and that the determining apparatus 101 has already determined that the type of bond between the ring 801 and each of the atoms bonded with the ring 801. Because $C\_(8\_7)$ is included in the ring 801, the determining apparatus 101 determines whether the type of bond between the ring 801 and each of the atoms bonded with the ring 801 is a single bond or a coordinate bond. Among the types of bonds having contact with the ring 801, the type of bond between $C\_(8\_3)$ and $C\_(8\_9)$ is a double bond and similarly, a bond is present that is not a single bond or a coordinate bond. Therefore, the determining apparatus 101 determines that the type of bond between the atoms of the ring 801 is not an aromatic bond. The type of bond between atoms of the ring 801 is determined by the method executed using electron density described with reference to FIG. 7.

It is assumed that, in FIG. 8B1, the determining apparatus 101 determines that the type of bond between $H\_(8\_11)$ and $C\_(8\_12)$ is a single bond. The determining apparatus 101 determines whether any one among $H\_(8\_11)$ and $C\_(8\_12)$ is included in the ring. In the case of FIG. 8B1, $C\_(8\_12)$ is included in the ring because $C\_(8\_12)$ to $C\_(8\_17)$ form the ring. Hereinafter, the ring formed by $C\_(8\_12)$ to $C\_(8\_17)$ will be referred to as "ring 802". At the stage depicted in FIG. 8B1, the types of bonds between the ring 802 and each of the atoms bonded with the ring 802 remain undetermined and therefore, the determining apparatus 101 does not determines whether the type of bond between the atoms included in the ring 802 is an aromatic bond.

It is assumed that, FIG. 8B2, the determining apparatus 101 determines that the type of bond between $C\_(8\_17)$ and $H\_(8\_18)$ is a single bond. Because $C\_(8\_17)$ is included in the ring 802, the determining apparatus 101 determines whether the type of bond between the ring 802 and each of the atoms bonded with the ring 802 is a single bond or a coordinate bond. Because the type of bond between the ring 802 and each of the atoms bonded with the ring 802 is a single bond, the determining apparatus 101 determines that the type of bond between the atoms included in the ring 802 is an aromatic bond.

FIGs. 9A and 9B are explanatory diagrams of an example of the determination method for a bond type having a charge and executed using electron density. The value of the electron density of the single bond including an anionic atom is higher than that of a single bond between neutral atoms, and may exceed the threshold value of 1.5 for judging whether the bond is a single bond or a double bond. A method of determining whether a single bond includes an anionic atom, executed when the value of the electron density exceeds the threshold value of 1.5 will be described with reference to FIGs. 9A and 9B.

The determining apparatus 101 extracts from the combinations of atoms in $C_{12}H_6O_4$, a combination whose bond type is a double bond. In FIG. 9A, the determining apparatus 101 extracts combinations of $C\_(9\_1)$ and $O\_(9\_2)$, $C\_(9\_3)$ and $O\_(9\_4)$, $C\_(9\_5)$ and $O\_(9\_6)$, and $C\_(9\_7)$ and $O\_(9\_8)$. It is assumed that the values of the electron densities $\rho$ of $C\_(9\_1)$ and $O\_(9\_2)$, $C\_(9\_3)$ and $O\_(9\_4)$, $C\_(9\_5)$ and $0\_(9\_6)$, and $C\_(9\_7)$ and $O\_(9\_8)$ are respectively 1.51, 1.72, 1.71, and 1.50.

All four combinations extracted are respectively a combination of a carbon atom and an oxygen atom and therefore, the determining apparatus 101 continues to execute the process for the four combinations. If the two atoms in one of the combinations extracted differ from those of the other combinations, the determining apparatus 101 divides the combinations into groups according to the two atomic species in the combinations and continues to execute the process for each of the groups, separately. For example, when the combinations extracted include combinations of two carbon atoms and combinations of a carbon atom and an oxygen atom, the determining apparatus 101 sets the combinations of the two carbon atoms to be one group, and sets the combinations of the carbon atom and the oxygen atom to be another group.

The determining apparatus 101 determines whether the values of the electron densities of the four combinations extracted are equal to each other. In the determination, if the values of the electron densities are not completely equal and for example, the difference is within a predetermined threshold value, in the value of the electron density between the combinations to be compared, the values may be regarded as equal. In the example of FIG. 9A, when the difference in the value of the electron density between the combinations to be compared is less than 0.05, the values are regarded as equal. For example, the value of the electron density of C_(9_1) and O_(9_2) is 1.51 and that of C_(9_3) and O_ (9_4) is 1.72 and therefore, 1.72-1.51=0.21>0.05 holds and the determining apparatus 101 determines that the values are not equal. Similarly, the determining apparatus 101 determines that the values of the electron densities of C_(9_5) and 0_(9_6) and C_(9_7) and O_(9_8) are not equal to each other.

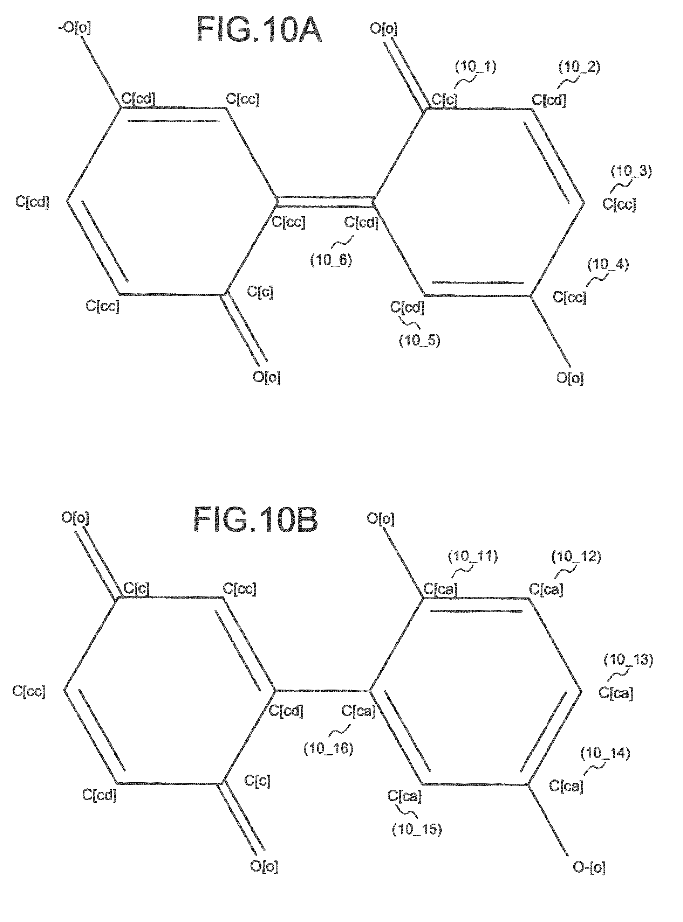

When the determining apparatus 101 determines that the values are not equal to each other, the determining apparatus 101 determines that the bond type whose electron density value is lower than that of the other is a single bond. FIG. 9B is a diagram of the state after the setting. The determining apparatus 101 determines that the type of bond between C_(9_1) and O_ (9_2) is the single bond and that O_(9_2) is O_(9_2). Similarly, the determining apparatus 101 determines that the type of bond between C_(9_7) and O_(9_8) is a single bond and that O_(9_8) is O⁻ _(9_8). An example of the determination of the atomic species executed using the bond type determined and depicted in FIGs. 7A to 9B will be described with reference to FIGs. 10A and 10B.

FIGs. 10A and 10B are explanatory diagrams of examples of the determination result of the atomic species. In FIGs. 10A and 10B, for $C_{12}H_6O_4$, the atomic species in the structure depicted in FIG. 7A is depicted in FIG. 10A and the atomic species in the structure depicted in FIG. 7B2 is depicted in FIG. 10B.

The atomic species can be identified one to one from the bond type. FIGs. 10A and 10B depict examples of a case where the GAFF atomic species is used. Although hydrogen atoms are not depicted in FIGS. 10A and 10B, the GAFF atomic species of each of the hydrogen atoms is [ha].

In FIG. 10A, the determining apparatus 101 determines that the GAFF atomic species of C_(10_1) to C_(10_6) are respectively [c], [cd], [cc], [cc], [cd], and [cd].

In FIG. 10B, the determining apparatus 101 determines that the GAFF atomic species of C_(10_1) to C_(10_6) are each [ca]. Because the difference in the GAFF species causes a difference in the molecular force field allocated, this affects the simulations of the physical properties, etc.

As depicted in FIGs. 10A and 10B, the difference in the atomic species causes a difference in the force field allocated and therefore, the simulations become inaccurate. The determining apparatus 101 according to the embodiment can allocate proper molecular force fields even to new molecules. An example will be described of a comparison between the calculation result of the electron density used in the determination of the bond type and the calculation result of the bonding distance used in the determination of the bond type in the conventional techniques.

FIG. 11 is an explanatory diagram of an example of the comparison between the calculation result of the electron density and the calculation result of the bonding distance. A table 1101 is a table that presents an example of comparison between the results of the calculation method for bond type determined by using electron density according to the embodiment, and the results acquired by the calculation method for bond type determined using the bonding distance. In FIG. 11, a comparison is executed between the calculation results of phosphorus atoms in two molecules of $P_2H_2$ whose phosphorus atoms are bonded by a double bond and $P_2H_4$ whose phosphorus atoms are bonded by a single bond, as the molecules to be compared. Hereinafter, $P_2H_2$ will be referred to as "molecule A" and $P_2H_4$ will be referred to as "molecule B".

In FIG. 11, the electron density and the bonding distance are calculated using an Austin model 1 (AM1) method and the PM5 method as the two calculation methods. Uses are present, each corresponding to a purpose of using each of the calculation methods, such as a calculation method used when the calculation result may be rough while the processing time period needs to be short, and a calculation method used when the processing time may be long while an accurate calculation result needs to be acquired.

For the molecule A, the determining apparatus 101 calculates the electron density $\rho$ between the P atoms to be 2.02 using the AM1 method and for the molecule B, calculates the electron density $\rho$ between the P atoms to be 1.01 also using the AM1 method. Thus, the threshold value for determining a double bond or a single bond when the AM1 method is used is determined to be 1.5, taking the mean value of the two values.

For the molecule A, the determining apparatus 101 calculates the electron density $\rho$ between the P atoms to be 2.03 using the PM5 method and for the molecule B, calculates the electron density $\rho$ between the P atoms to be 1.03 using the AM1 method. Thus, the threshold value of the electron density for determining a double bond or a single bond, used when the PM5 method is used is determined to be 1.5, taking the mean value of the two values. In this manner, when the electron density is used to determine the bond type, the determining apparatus 101 can use the same threshold value regardless of the calculation method.

For the molecule A, the determining apparatus 101 calculates the bonding distance between the P atoms to be 1.757 [angstrom] using the AM1 method and for the molecule B, calculates the bonding distance between the P atoms to be 1.990 [angstrom] also using the AM1 method. Thus, the threshold value for determining a double bond or a single bond, used when the AM1 method is used is determined to be 1.874 [angstrom], taking the mean value of the two values.

For the molecule A, the determining apparatus 101 calculates the bonding distance between the P atoms to be 1.889 [angstrom] using the PM5 method and for the molecule B, calculates the bonding distance between the P atoms to be 2.012 [angstrom] also using the PM5 method. Thus, the threshold value of the bonding distance for determining a double bond or a single bond, used when the PM5 method is used is determined as 1.951 [angstrom] taking the mean value of the two values. In this manner, when the bond type is determined using the bonding distance, the variation of the results of the calculation method is large and therefore, the determining apparatus 101 uses the threshold value according to the calculation method used.

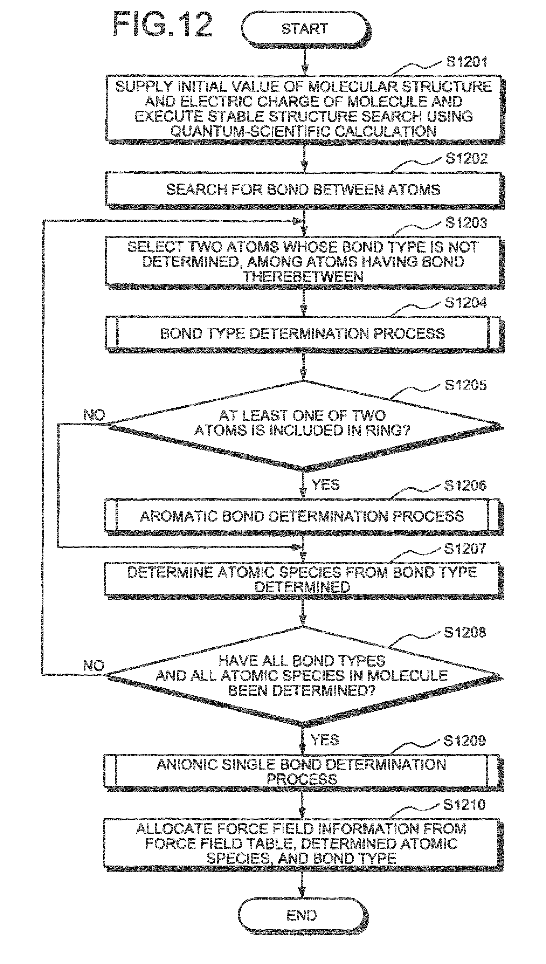

Therefore, when the bond type is determined using the bonding distance, the determining apparatus 101 needs to store therein a threshold value for each calculation. When the determining apparatus 101 uses in another calculation method, the threshold value used in a given calculation method, the bond type is wrongly determined. With reference to FIGs. 12 to 15, flowcharts will be described for determining the atomic species using electron density.

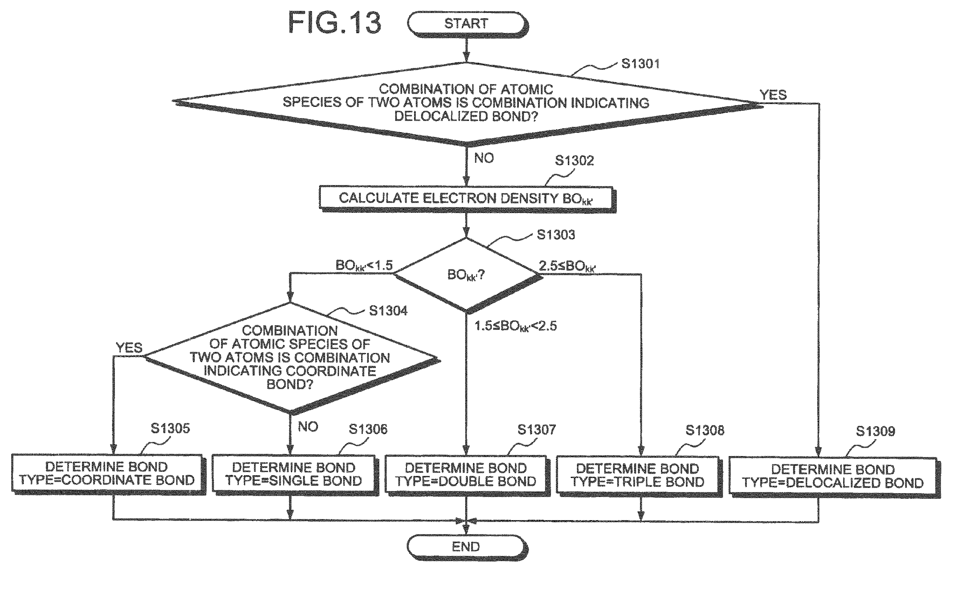

FIG. 12 is a flowchart of an example of a force field allocation process procedure. The force field allocation process is a process of allocating a force field to a new molecule. The determining apparatus 101 supplies the initial value of the molecular structure and the electric charge of the entire molecule and thereby, executes a stable structure search using a quantum-scientific calculation (step S1201). In the stable structure search, the electron density matrix P and the atomic orbital overlapping integral matrix S are calculated. The determining apparatus 101 searches for a bond between atoms (step S1202) and selects two atoms whose bond type is not determined, as the atoms having a bond therebetween (step S1203). The determining apparatus 101 executes a bond type determination process (step S1204). Details of the bond type determination process will be described later with reference to FIG. 13. The determining apparatus 101 determines whether at least one of the two atoms is included in a ring (step S1205).

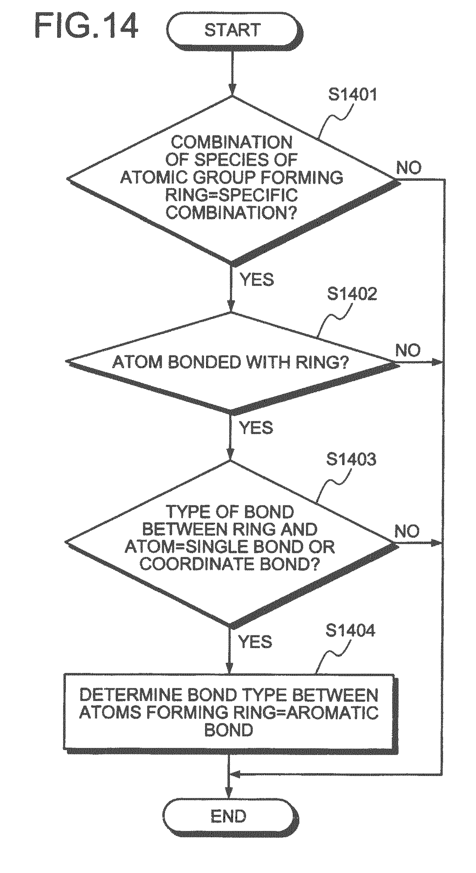

If the determining apparatus 101 determines that at least one of the two atoms is included in a ring (step S1205: YES), the determining apparatus 101 executes an aromatic bond determination process (step S1206). Details of the aromatic bond determination process will be described later with reference to FIG. 14. After the execution of the process at step S1206 or if the determining apparatus 101 determines that neither of the two atoms is included in a ring (step S1205: NO), the determining apparatus 101 determines the atomic species from the bond type determined (step S1207). The determining apparatus 101 determines whether all the bond types and all the atomic species in the molecule have been determined (step S1208). If the determining apparatus 101 determines that not all the bond types and not all the atomic species have been determined (step S1208: NO), the determining apparatus 101 progresses to a process at step S1203.

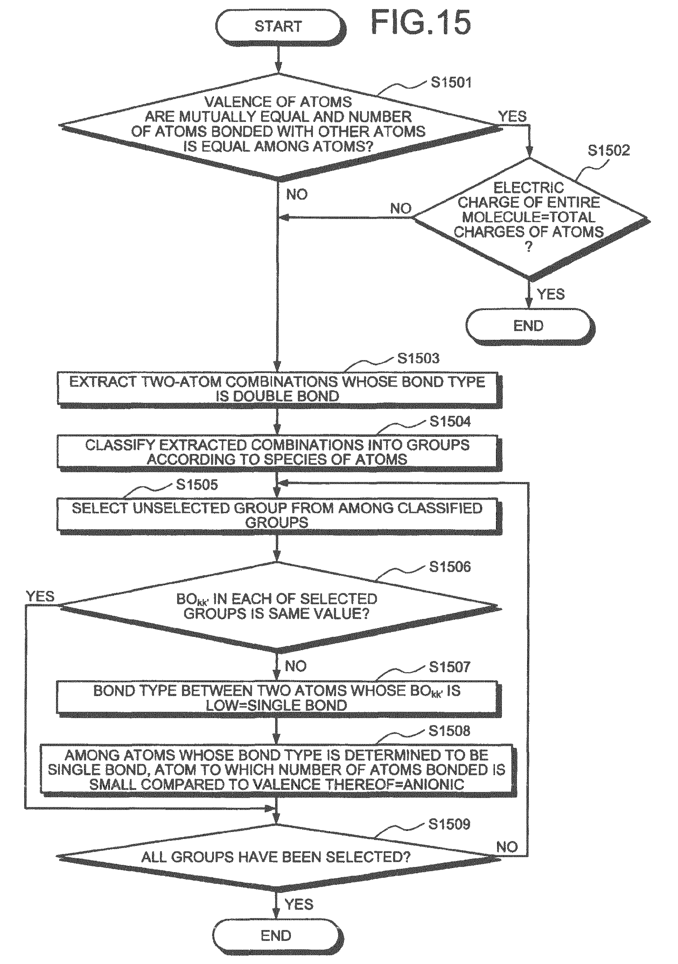

If the determining apparatus 101 determines that all the bond types and all the atomic species in the molecule have been determined (step S1208: YES), the determining apparatus 101 executes an anionic single bond determination process (step S1209). Details of the anionic single bond determination process will be described with reference to FIG. 15. The anionic single bond determination process is a process of searching for a single bond having therein an anionic atom. A single bond having therein an anionic atom is defined using the AM1BCC charge and is not defined in the GAFF force field. Therefore, when the GAFF force field is used, the determining apparatus 101 does not need to execute the process at step S1209.

After the execution of the process at step S1209, the determining apparatus 101 allocates force field information from the force field table, the determined atomic species and the bond type (step S1210). For example, the determining apparatus 101 may output the determined atomic species and the bond type to another apparatus at step S1210. After this output, the other apparatus may allocate the force field information that corresponds to the atomic species and the bond type determined. The force field table refers to, for example, a table that stores a spring constant for each of the atomic species described in the above Published Japanese-Translation of PCT Application, Publication No. 2008-041304WO. After step S1210 comes to an end, the determining apparatus 101 causes the force field allocation process to come to an end. The determining apparatus 101 can allocate a proper force field to a new molecule by executing the force field allocation process.

FIG. 13 is a flowchart of an example of the bond type determination process procedure. The bond type determination process procedure is a process of determining a bond type. The determining apparatus 101 determines whether the combination of the atomic species of the two atoms is a combination indicating a delocalized bond (step S1301). If the determining apparatus 101 determines that the combination is not a combination indicating a delocalized bond (step S1301: NO), the determining apparatus 101 calculates the electron density $BO_{kk'}$ (step S1302). For the process at step S1302, the amount of processing to calculate the electron density is negligibly small compared to that of step S1201 because the electron density matrix P and the atomic orbital overlapping integral matrix S are calculated in the process at step S1201.

The determining apparatus 101 checks the electron density $BO_{kk'}$ between the two atoms (step S1303) . If the electron density $BO_{kk'}$ is lower than 1.5 (step S1303: $BO_{kk'}$<1.5), the determining apparatus 101 determines whether the combination of the atomic species of the two atoms is a combination indicating a coordinate bond (step S1304) . In the process at step S1304, the coordinate bond is defined using the AM1BCC charge and is not defined in the GAFF force field. Therefore, when the GAFF force field is used, the determining apparatus 101 does not execute the process at step S1304 and progresses to the process at step S1306. If the determining apparatus 101 determines that the combination is a combination indicating a coordinate bond (step S1304: YES), the determining apparatus 101 determines that the bond type is a coordinate bond (step S1305) .

If the determining apparatus 101 determines that the combination is not a combination indicating a coordinate bond (step S1304: NO), the determining apparatus 101 determines that the bond type is a single bond (step S1306) . If $BO_{kk'}$ is greater than or equal to 1.5 and less than 2.5 (step S1303: $1.5 \leq BO_{kk'} < 2.5$), the determining apparatus 101 determines that the bond type is a double bond (step S1307) . If $BO_{kk'}$ is greater than or equal to 2.5 (step S1303: $2.5 \leq BO_{kk'}$), the determining apparatus 101 determines that the bond type is a triple bond (step S1308) . If the determining apparatus 101 determines that the combination is a combination indicating a delocalized bond (step S1301: YES), the determining apparatus 101 determines that the bond type is a delocalized bond (step S1309) . After the process at any one of steps S1305 to S1309 comes to an end, the determining apparatus 101 causes the bond type determination process to come to an end. The determining apparatus 101 can determine properly the bond type by executing the bond type determination process.

FIG. 14 is a flowchart of an example of an aromatic bond determination process procedure. An aromatic bond determination process is a process of determining the bond type to be an aromatic bond. The determining apparatus 101 determines whether the combination of species of the atomic group forming the ring is a specific combination (step S1401). If the determining apparatus 101 determines that the combination is a specific combination (step S1401: YES), the determining apparatus 101 determines whether an atom is present that is bonded with the ring (step S1402). If the determining apparatus 101 determines that an atom is present that is bonded with the ring (step S1402: YES), the determining apparatus 101 determines if the type of bond between the ring and the atom bonded with the ring is a single bond or a coordinate bond (step S1403). If the determining apparatus 101 determines that the type of bond is a single bond or a coordinate bond (step S1403: YES), the determining apparatus 101 determines that the bond type between the atoms forming the ring is an aromatic bond (step S1404).

After the step S1403 comes to an end, if the determining apparatus 101 determines that the combination is not a specific combination (step S1401: NO), if the determining apparatus 101 determines that no atom is present that is bonded with the ring (step S1402: NO), or if the determining apparatus 101 determines that the type of bond is neither a single bond nor a coordinate bond (step S1403: NO), the determining apparatus 101 causes the aromatic bond determination process to come to an end. The determining apparatus 101 can determine properly the bond type for the atom group that is bonded by an aromatic bond by executing the aromatic bond determination process.

FIG. 15 is a flowchart of an example of an anionic single bond determination process procedure. An anionic single bond determination process is a process of searching for a bond that may be a single bond including an anionic atom and when the condition is satisfied, determining the bond type to be a single bond that includes an anionic atom. The determining apparatus 101 determines whether the valences of the atoms are mutually equal and the number of atoms bonded with other atoms is equal among the atoms (step S1501). If the determining apparatus 101 determines that the valences are all equal and the number of atoms bonded are equal among the atoms (step S1501: YES), the determining apparatus 101 determines whether the electric charge of the entire molecule and the total of the charges of the atoms are equal to each other (step S1502). If the determining apparatus 101 determines that the electric charge of the entire molecule and the total of the charges of the atoms are equal to each other (step S1502: YES), the determining apparatus 101 causes the anionic single bond determination process to come to an end.

If the determining apparatus 101 determines that the valences are not equal and the number of atoms bonded is not equal (step S1501: NO) or if the determining apparatus 101 determines that the electric charge of the entire molecule and the total of the charges of the atoms are not equal (step S1502: NO), the determining apparatus 101 extracts combinations whose bond type is a double bond in the molecule (step S1503) and classifies the extracted combinations into groups according to the species of the two atoms (step S1504). The determining apparatus 101 selects unselected groups among the classified groups (step S1505). If one combination is included in the group selected, the determining apparatus 101 progresses to the process at step S1509 described later. The determining apparatus 101 determines whether BOkk' in each of the groups selected is the same value (step S1506).

If the determining apparatus 101 determines that BOkk' in each of the groups selected is not the same value (step S1506: NO), the determining apparatus 101 determines that the bond type between the two atoms whose BOkk' low is a single bond (step S1507). The determining apparatus 101 determines that among atoms whose bond type is determined to be a single bond, an atom to which the number of atoms bonded is small compared to the valence of the atom is anionic (step S1508).

When the process at step S1508 comes to an end or if the determining apparatus 101 determines that BOkk' in each of the groups selected is the same value (step S1506: YES), the determining apparatus 101 determines whether all the groups have been selected (step S1509). If the determining apparatus 101 determines that an unselected group is present (step S1509: NO), the determining apparatus 101 progresses to the process at step S1505. If the determining apparatus 101 determines that all the groups have been selected (step S1509: YES), the determining apparatus 101 causes the anionic single bond determination process to come to an end. The determining apparatus 101 can more correctly determine the bond type and therefore, can more accurately allocate molecular force fields by executing the anionic single bond determination process.

As described, according to the determining apparatus 101, the determining apparatus 101 determines the atomic species of two atoms using the electron density of the quantum-scientific calculation. Thereby, the determining apparatus 101 can determine the atomic species using the same threshold value regardless of the calculation method of the quantum-scientific calculation and therefore, the accuracy in determining the bond type between atoms can be improved. The force field allocated becomes more realistic and therefore, simulations for new molecules can accurately be executed in the chemical industry and, especially, in the pharmaceutical industry. The same threshold value can be used regardless of the calculation method of the quantum-scientific calculation and therefore, the determining apparatus 101 does not need to store any data that indicates which calculation method is used for the quantum-scientific calculation.

With the method of determining the bond type using the bonding distance, the initial parameter used to calculate the bonding distance differs depending on the calculation method such as the AM1 method or the PM5 method. Therefore, the value of the calculation result also differs and as a result, the threshold value also differs depending on the calculation method. On the other hand, with the method of determining the bond type using electron density, the initial parameter used to calculate the electron density is the number of electrons. Therefore, all the calculation methods employ the same initial parameter and the calculation results thereof yield values that are close to each other. As a result, the same threshold value can be used regardless of the calculation method.

According to the determining apparatus 101, the bond type may be determined using the first condition for the electron density between atoms bonded by the single bond, the second condition for the electron density between atoms bonded by a double bond, and the third condition for the electron density between atoms bonded by a triple bond. Thereby, the determining apparatus 101 can determine each of the bond types from a single bond to a third bond using the electron density, and also can allocate a force field that corresponds to each of the bond types from a single bond to a third bond.

According to the determining apparatus 101, the determining apparatus 101 may determine the bond type to be a coordinate bond using the fourth condition for the electron density between atoms bonded by a coordinate bond and the fifth condition for the atomic species of atoms bonded by a coordinate bond. Thereby, the determining apparatus 101 can allocate a force field that corresponds to a coordinate bond.

According to the determining apparatus 101, the determining apparatus 101 may determine the bond type to be an aromatic bond using the sixth condition for the species of atoms capable of forming a ring that is formed by an atom group bonded by an aromatic bond. Thereby, the determining apparatus 101 can allocate a force field that corresponds to an aromatic bond.

According to the determining apparatus 101, the determining apparatus 101 may determine the bond type to be an aromatic bond using the sixth condition, and the seventh condition for the type of bond between a ring formed by an atom group bonded by an aromatic bond and atoms bonded with the ring. Thereby, the determining apparatus 101 can allocate a force field that corresponds to the aromatic bond. Using the sixth and the seventh conditions improves the accuracy in assessing the bond type and therefore, the determining apparatus 101 can improve the accuracy of the simulation results.

According to the determining apparatus 101, when the determining apparatus 101 determines that the type of bond between two atoms is a double bond, the determining apparatus 101 may determine that the bond type is an anionic single bond, by comparing the electron density between the two atoms and the electron density between other atoms in the molecule bonded by a double bond. Thereby, the accuracy in assessing the bond type is improved and the determining apparatus 101 can improve the accuracy of the simulation result.

The determining method described in the present embodiment may be implemented by executing a prepared program on a computer such as a personal computer and a workstation. The program is stored on a computer-readable recording medium such as a hard disk, a flexible disk, a CD-ROM, an MO, and a DVD, read out from the computer-readable medium, and executed by the computer. The program may be distributed through a network such as the Internet.

All examples and conditional language provided herein are intended for pedagogical purposes of aiding the reader in understanding the invention and the concepts contributed by the inventor to further the art, and are not to be construed as limitations to such specifically recited examples and conditions, nor does the organization of such examples in the specification relate to a showing of the superiority and inferiority of the invention. Although one or more embodiments of the present invention have been described in detail, it should be understood that the various changes, substitutions, and alterations could be made hereto without departing from the spirit and scope of the invention.

## Claims

1. A determination program causing a computer to execute a process comprising:

   calculating by referring to a first storing unit storing an electron density of electrons belonging to each atom in a molecule and a degree of overlap of atomic orbitals between the atoms in the molecule, an electron density between a first atom and a second atom different from the first atom respectively selected from the molecule in a structurally stable state;
   determining a bond type of a bond between the first and the second atoms, based on the calculated electron density and by referring to a second storing unit correlating and storing bond types representing types of bonds between atoms, and conditions for the electron density between atoms for each bond type; and
   outputting the determined bond type between the first and the second atoms.

2. The determination program according to claim 1, wherein
   the second storing unit correlates and stores a single bond as a bond type and a first condition for the electron density between atoms bonded by the single bond, a double bond as a bond type and a second condition for the electron density between atoms bonded by the double bond, and a triple bond as a bond type and a third condition for the electron density between atoms bonded by the triple bond,
   the process further comprises assessing whether the calculated electron density satisfies any among the first, the second, and the third conditions stored in the second storing unit, and
   the determining includes determining the type of bond between the first and the second atoms to be the type of bond correlated with the condition that is assessed to be satisfied by the calculated electron density, when the calculated electron density is assessed to satisfy any one among the first, the second, and the third conditions.

3. The determination program according to claim 1 or 2, wherein
   the second storing unit correlates and stores a coordinate bond as a bond type and a fourth condition for the electron density between atoms bonded by the coordinate bond, and further correlates and stores the coordinate bond and a fifth condition for a combination of atomic species of atoms bonded by the coordinate bond,
   the process further comprises:

   assessing whether the electron density calculated satisfies the fourth condition stored in the second storing unit; and
   assessing whether a combination of atomic species of the first atom and atomic species of the second atom satisfies the fifth condition stored in the second storing unit, and

   the determining includes determining the type of bond between the first and the second atoms to be the coordinate bond, when the fourth and the fifth conditions are assessed to be satisfied.

4. The determination program according to any one of claims 1 to 3, wherein
   the second storing unit correlates and stores an aromatic bond as a bond type and a sixth condition for an atomic species capable being bonded by the aromatic bond and forming a ring, and
   the process comprises:

   assessing whether in the molecule, an atom group is present that forms a ring;
   assessing, when an atom group that forms a ring is assessed to be present, whether atomic species of each atom of the atom group satisfies the sixth condition; and
   determining the type of bond between the atoms of the atom group forming the ring is the aromatic bond, when the sixth condition is assess to be satisfied.

5. The determination program according to claim 4, wherein
   the second storing unit further correlates and stores the aromatic bond and a seventh condition for a bond type between the ring formed by the atoms bonded by the aromatic bond and an atom bonded to the ring, and
   the process comprises:

   assessing whether in the molecule, an atom is present that is bonded with the ring, when the type of bond between the first and the second atoms is determined and in the molecule, an atom group forming a ring is assessed to be is present;
   assessing, when an atom is present that is bonded with the ring, whether the bond type between the ring and the atom bonded with the ring satisfies the seventh condition stored in the second storing unit; and
   determining the bond type between the atoms of the atom group forming the ring to be the aromatic bond, when the seventh condition is assessed to be satisfied.

6. The determination program according to any one of claims 2 to 5, the process comprising:

extracting from among the atoms in the molecule, remaining after excluding the first and the second atoms and when the bond type between the first and the second atoms is the double bond, a third atom and a fourth atom having a bond type is the double bond and of an atomic species combination identical to the atomic species combination of the first and the second atoms;

judging whether the electron density between the first and the second atoms is equal to the electron density between the third and the fourth atoms; and

determining, when the electron density between the first and the second atoms is judged to not be equal to the electron density between the third and the fourth atoms, the bond type of the electron density that is lower among the electron density between the first and the second atoms and the electron density between the third and the fourth atoms to be the single bond, and determining to be an anionic atom, any one among two atoms of a bond type determined to be the single bond.

7. A determination apparatus comprising:

a calculating unit (302) that by referring to a first storing unit storing an electron density of electrons belonging to each atom in a molecule and a degree of overlap of atomic orbitals between the atoms in the molecule, calculates an electron density between a first atom and a second atom different from the first atom respectively selected from the molecule in a structurally stable state;

a determining unit (304) that determines a bond type of a bond between the first and the second atoms, based on the calculated electron density and by referring to a second storing unit correlating and storing bond types representing types of bonds between atoms, and conditions for the electron density between atoms for each bond type; and

an output unit (307) that outputs the determined bond type between the first and the second atoms.

8. A determination method executed by a computer, the determination process comprising:

calculating by referring to a first storing unit storing an electron density of electrons belonging to each atom in a molecule and a degree of overlap of atomic orbitals between the atoms in the molecule, an electron density between a first atom and a second atom different from the first atom respectively selected from the molecule in a structurally stable state;

determining a bond type of a bond between the first and the second atoms, based on the calculated electron density and by referring to a second storing unit correlating and storing bond types representing types of bonds between atoms, and conditions for the electron density between atoms for each bond type; and

outputting the determined bond type between the first and the second atoms.

FIG.1

# FIG.2

EP 2 645 279 A2

EP 2 645 279 A2

**FIG.3**

# FIG.4

312

| | BOND TYPE | ELECTRON DENSITY CONDITION | ATOM CONDITION | OTHER CONDITIONS | FORCE FIELD TYPE |
|---|---|---|---|---|---|
| 401-1 | SINGLE BOND | ELECTRON DENSITY<1.5 | NONE | NONE | AM1BCC CHARGE GAFF FORCE FIELD |
| 401-2 | DOUBLE BOND | 1.5≤ELECTRON DENSITY<2.5 | NONE | NONE | AM1BCC CHARGE GAFF FORCE FIELD |
| 401-3 | TRIPLE BOND | 2.5≤ELECTRON DENSITY | NONE | NONE | AM1BCC CHARGE GAFF FORCE FIELD |
| 401-4 | COORDI-NATE BOND | ELECTRON DENSITY<1.5 | NITROGEN ATOM AND OXYGEN ATOM (N-oxide) OR NITROGEN ATOM AND SULFUR ATOM (N-sulfide) OR ... | NONE | AM1BCC CHARGE |
| 401-5 | AROMA-TIC BOND | BOND IS PRESENT | ANY ONE AMONG SIX ATOMS IS ATOMIC SPECIES OF CARBON ATOM, NITROGEN ATOM, ..., DICATIONIC SULFUR ATOM? OR TWO ADJACENT ATOMS AMONG SIX ATOMS ARE BONDED BY AROMATIC BOND OF SIX-MEMBERED RING AND ANY ONE OF AMONG OTHER FOUR ATOMS IS ATOMIC SPECIES OF CARBON ATOM, NITROGEN ATOM, ..., DICATIONIC SULFUR ATOM? OR ... | BOND TYPE WITH ATOM BONDED WITH RING IS SINGLE BOND OR COORDINATE BOND | AM1BCC CHARGE GAFF FORCE FIELD |
| 401-6 | DELO-CALIZED BOND | BOND IS PRESENT | NITROGEN ATOM AND OXYGEN ATOM IN NITRO GROUP OR CARBON ATOM AND OXYGEN ATOM IN CARBOXYLATE ION OR PHOSPHOROUS ATOM AND OXYGEN ATOM IN PHOSPHATE ION OR SULFUR ATOM AND OXYGEN ATOM IN SULFORATE ION OR ... | NONE | AM1BCC CHARGE |

EP 2 645 279 A2

# FIG.5A

MOLECULAR STRUCTURE TABLE — 501

| ATOM ID | x COOR-DINATE | y COOR-DINATE | z COOR-DINATE |
|---|---|---|---|
| C_(5_1) | 0.66 | 0.0 | 0.0 |
| C_(5_2) | -0.66 | 0.0 | 0.0 |
| H_(5_3) | 0.8 | 0.4 | 0.0 |
| H_(5_4) | -0.8 | 0.4 | 0.0 |
| H_(5_5) | 0.8 | -0.4 | 0.0 |
| H_(5_6) | -0.8 | -0.4 | 0.0 |

501-1, 501-2, 501-3, 501-4, 501-5, 501-6

# FIG.5B

EP 2 645 279 A2

FIG.6A

FIG.6B

(6_1)

(6_3)

(6_2)

(6_4)

FIG.6C

(6_5)

(6_7)

2-

(6_6)

(6_8)

FIG.7A

FIG.7B1

FIG.7B2

FIG.8A1

FIG.8B1

FIG.8A2
NOT DETERMINED AS
AROMATIC BOND

FIG.8B2
DETERMINED AS
AROMATIC BOND

EP 2 645 279 A2

FIG.9A

FIG.9B

# FIG.10A

-O[o]

C[cd]    C[cc]

C[cd]

C[cc]

C[c]

O[o]

O[o]

C[c] (10_1)    C[cd] (10_2)

C[cc] (10_3)

C[cc] (10_4)

O[o]

C[cd] (10_5)

C[cc]    C[cd] (10_6)

# FIG.10B

O[o]

C[c]    C[cc]

C[cc]

C[cd]

C[c]

O[o]

O[o]

C[ca] (10_11)    C[ca] (10_12)

C[ca] (10_13)

C[ca] (10_14)

O-[o]

C[ca] (10_15)

C[cd]    C[ca] (10_16)

# FIG.11

| MOLECULE | DETERMINATION USING ELECTRON DENSITY | | DETERMINATION USING BONDING DISTANCE | |
|---|---|---|---|---|
| | AM1 METHOD | PM5 METHOD | AM1 METHOD | PM5 METHOD |
| A: H-P=P-H<br><br>B: H₂P-PH₂ | ρ(P=P)=2.02<br>→ THRESHOLD VALUE: 1.5<br>ρ(P-P)=1.01 | 2.03<br>→ 1.5<br>1.03 | r(P=P)=1.757<br>→ THRESHOLD VALUE: 1.874<br>r(P-P)=1.990 | 1.889<br>→ 1.951<br>2.012 |

## FIG.12

```
START
```

S1201

SUPPLY INITIAL VALUE OF MOLECULAR STRUCTURE AND ELECTRIC CHARGE OF MOLECULE AND EXECUTE STABLE STRUCTURE SEARCH USING QUANTUM-SCIENTIFIC CALCULATION

S1202

SEARCH FOR BOND BETWEEN ATOMS

S1203

SELECT TWO ATOMS WHOSE BOND TYPE IS NOT DETERMINED, AMONG ATOMS HAVING BOND THEREBETWEEN

S1204

BOND TYPE DETERMINATION PROCESS

S1205

AT LEAST ONE OF TWO ATOMS IS INCLUDED IN RING?

NO

YES          S1206

AROMATIC BOND DETERMINATION PROCESS

S1207

DETERMINE ATOMIC SPECIES FROM BOND TYPE DETERMINED

S1208

HAVE ALL BOND TYPES AND ALL ATOMIC SPECIES IN MOLECULE BEEN DETERMINED?

NO

YES          S1209

ANIONIC SINGLE BOND DETERMINATION PROCESS

S1210

ALLOCATE FORCE FIELD INFORMATION FROM FORCE FIELD TABLE, DETERMINED ATOMIC SPECIES, AND BOND TYPE

```
END
```

FIG.13

START

S1301

COMBINATION OF ATOMIC
SPECIES OF TWO ATOMS IS COMBINATION INDICATING
DELOCALIZED BOND?

YES

NO

S1302

CALCULATE ELECTRON DENSITY $BO_{kk'}$

S1303

$BO_{kk'}<1.5$          $BO_{kk'}$?          $2.5 \leq BO_{kk'}$

$1.5 \leq BO_{kk'} < 2.5$

S1304

COMBINATION
OF ATOMIC SPECIES OF
TWO ATOMS IS COMBINATION
INDICATING COORDINATE
BOND?

YES

NO

S1305          S1306          S1307          S1308          S1309

DETERMINE BOND
TYPE=COORDINATE BOND

DETERMINE BOND
TYPE=SINGLE BOND

DETERMINE BOND
TYPE=DOUBLE BOND

DETERMINE BOND
TYPE=TRIPLE BOND

DETERMINE BOND
TYPE=DELOCALIZED BOND

END

**FIG.14**

START

S1401

COMBINATION OF SPECIES OF ATOMIC GROUP FORMING RING=SPECIFIC COMBINATION? — NO

YES

S1402

ATOM BONDED WITH RING? — NO

YES

S1403

TYPE OF BOND BETWEEN RING AND ATOM=SINGLE BOND OR COORDINATE BOND? — NO

YES

S1404

DETERMINE BOND TYPE BETWEEN ATOMS FORMING RING=AROMATIC BOND

END

# FIG.15

START

S1501

VALENCE OF ATOMS ARE MUTUALLY EQUAL AND NUMBER OF ATOMS BONDED WITH OTHER ATOMS IS EQUAL AMONG ATOMS? — YES

NO

S1502

ELECTRIC CHARGE OF ENTIRE MOLECULE=TOTAL CHARGES OF ATOMS ?

NO

YES

END

S1503
EXTRACT TWO-ATOM COMBINATIONS WHOSE BOND TYPE IS DOUBLE BOND

S1504
CLASSIFY EXTRACTED COMBINATIONS INTO GROUPS ACCORDING TO SPECIES OF ATOMS

S1505
SELECT UNSELECTED GROUP FROM AMONG CLASSIFIED GROUPS

S1506
YES — $BO_{kk'}$ IN EACH OF SELECTED GROUPS IS SAME VALUE?

NO

S1507
BOND TYPE BETWEEN TWO ATOMS WHOSE $BO_{kk'}$ IS LOW=SINGLE BOND

S1508
AMONG ATOMS WHOSE BOND TYPE IS DETERMINED TO BE SINGLE BOND, ATOM TO WHICH NUMBER OF ATOMS BONDED IS SMALL COMPARED TO VALENCE THEREOF=ANIONIC

S1509
ALL GROUPS HAVE BEEN SELECTED? — NO

YES

END

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP H07282096 B
- JP 2006282929 A
- WO 2008041304 PCT
- WO 2008041304 A

### Non-patent literature cited in the description

- **WANG ; JUNMEI et al.** Automatic Atom Type and Bond Type Perception in Molecular Mechanical Calculations. *Journal of Molecular Graphics and Modeling,* 2006, vol. 25, 247-260
- **FUJITANI, HIDEAKI et al.** Massively Parallel Computation of Absolute Binding Free Energy with Well-Equilibrated States. *Physical Review E,* 2009, vol. 79, 021914
- **JAKALIAN ; ARAZ et al.** Fast, Efficient Generation of High-Quality Atomic Charge, AM1-BCC Model: II, Parameterization and Validation. *Journal of Computational Chemistry,* 2002, vol. 23, 1623-1641
- **JUNMEI et al.** Development and Testing of a General Amber Force Field. *Journal of Computational Chemistry,* 2004, vol. 25, 1157-1174
- **CORNELL, WENDY D. et al.** A Second Generation Force Field for the Simulation of Proteins, Nucleic Acids, and Organic Molecules. *Journals-American Chemical Society,* 1995, vol. 117, 5179-5197
- **MAYER, I.** Charge, Bond Order and Valence in the ab initio SCF Theory. *Chemical Physics Letters,* 1983, vol. 97, 270-274
- **KALINOWSKI, JAROSLAW A. et al.** Class IV Charge Model for the Self-Consistent Charge Density-Functional Tight-Binding Method. *Journal of Physical Chemistry A,* 2004, vol. 108, 2545-2549